(19) **Europäisches Patentamt European Patent Office Office européen des brevets**

(11) **EP 4 767 940 A1**

(12) **EUROPEAN PATENT APPLICATION**
published in accordance with Art. 153(4) EPC

(43) Date of publication:
01.07.2026 Bulletin 2026/27

(21) Application number: 25846667.1

(22) Date of filing: 27.05.2025

(51) International Patent Classification (IPC):
**A61B 5/243** (2021.01)    **A61B 5/00** (2006.01)

(52) Cooperative Patent Classification (CPC):
**A61B 5/00; A61B 5/243**

(86) International application number:
**PCT/CN2025/097299**

(87) International publication number:
**WO 2026/026175 (05.02.2026 Gazette 2026/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 02.08.2024 CN 202411058622

(71) Applicant: Nanjing Raygen Health Co., Ltd.
Nanjing, Jiangsu 210031 (CN)

(72) Inventors:
• ZHAO, Ting
Nanjing 210031 (CN)
• WEI, Caoliang
Nanjing, Jiangsu 210031 (CN)

(74) Representative: Dilg, Haeusler, Schindelmann
Patentanwaltsgesellschaft mbH
Leonrodstraße 58
80636 München (DE)

(54) **BIOMAGNETIC SIGNAL-BASED FOUR-DIMENSIONAL CARDIAC FUNCTION IMAGING METHOD AND DEVICE**

(57) Provided in the present application are a biomagnetic signal-based four-dimensional cardiac function imaging method and device. The method comprises: acquiring a cardiac magnetic signal of a subject to be tested and dynamic structure information of a target torso part within a full cardiac cycle, wherein the target torso part at least comprises two lungs, a torso, and a heart chamber and a pericardium structure thereof, and the dynamic structure information comprises a contour structure and a plurality of frames of dynamic images; performing spatial registration on the dynamic structure information and a sensor acquiring the cardiac magnetic signal to obtain coordinates of the sensor in a coordinate system of the dynamic structure information; calculating and reconstructing a cardiac electrical excitation activity based on the dynamic structure information, the coordinates of the sensor, and the cardiac magnetic signal to obtain a calculation and reconstruction result; and converting the calculation and reconstruction result into a four-dimensional visualization image.

FIG. 1

**Description**

CROSS-REFERENCE TO RELATED APPLICATIONS

The present application claims priority to Chinese patent application No. 202411058622X, filed on August 2, 2024, entitled "Biomagnetic Signal-based Four-Dimensional Cardiac Function Imaging Method and Device", which is hereby incorporated by reference in its entirety.

FIELD

The present application relates to the field of cardiac imaging, and in particular to a method and an apparatus for four-dimensional cardiac function imaging based on biomagnetic signals.

BACKGROUND

In modeling of non-invasive magnetocardiography imaging, traditional methods achieve non-invasive imaging and visualization of cardiac electrical excitation activity by measuring weak biological magnetic signals. In these traditional methods, a heart is often treated as a static object. While considering that opening of an aortic valve and ventricular ejection primarily occur after QRS complexes, treating the heart as the static object during the cardiac activation phase has some merit. However, this assumption of a static heart becomes inaccurate when a cardiac repolarization process is analyzed. This is because cardiac systolic activity mainly occurs near a T wave, especially because diagnostic criteria for myocardial ischemia or infarction are primarily manifested in an ST segment and a T wave. Furthermore, the reconstruction of unknown cardiac excitation activity using a limited number of sensor signals is defective, and even minor changes in the geometry of the heart or trunk can lead to errors in the reconstruction results.

On the other hand, traditional non-invasive cardiac imaging techniques mainly focus on the reconstruction of cardiac surfaces (including epicardium and endocardium), lacking precise characterization and imaging of depth information of intracardiac origins and intramural activation delay. It limits its application efficacy in the diagnosis of complex cardiac diseases such as ventricular arrhythmias to some extent.

Therefore, traditional technologies have problems of insufficient integrity and accuracy of cardiac imaging.

BRIEF SUMMARY

The present application provides a method and an apparatus for four-dimensional cardiac function imaging based on biomagnetic signals to solve problems of insufficient integrity and accuracy of cardiac imaging in the related art, and to achieve complete and accurate cardiac imaging.

The present application provides a method for four-dimensional cardiac function imaging based on biomagnetic signals, including:

acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images;

performing spatial registration on the dynamic structural information and sensors acquiring the cardiac magnetic signals to obtain coordinates of the sensors in a coordinate system for the dynamic structural information;

performing calculation and reconstruction on cardiac electrical excitation activity based on the dynamic structural information, the coordinates of the sensors, and the cardiac magnetic signals to obtain calculation and reconstruction results; and

converting the calculation and reconstruction results into a four-dimensional visual image.

According to the method for four-dimensional cardiac function imaging based on the biomagnetic signals provided in the present application, performing the calculation and reconstruction on cardiac electrical excitation activity based on the dynamic structural information, the coordinates of the sensors, and the cardiac magnetic signals to obtain the calculation and reconstruction results includes:

generating a dynamic distributed cardiac equivalent source model during a full cardiac cycle based on the plurality of frames of dynamic images;

performing calculation based on the dynamic distributed cardiac equivalent source model, the coordinates of the sensor, and the contour structure to obtain a dynamic lead field matrix; and

performing calculation and reconstruction on cardiac electrical excitation activity based on the cardiac magnetic signals and the dynamic lead field matrix to obtain the calculation and reconstruction results.

According to the method for four-dimensional cardiac function imaging based on the biomagnetic signals provided in the present application, performing the spatial registration on the dynamic structural information and the sensors acquiring the cardiac magnetic signals to obtain the coordinates of the sensors in the coordinate system for the dynamic structural information includes:

marking a first spatial coordinate of each body surface contrast marker in the coordinate system for the dynamic structural information, and recording a second spatial coordinate of each cardiac magnetic measurement marker in a digital coordinate system using a digital measurement device; where body surface contrast markers are a preset number of markers set on a trunk of the subject during acquisition of the dynamic structural information, and the cardiac magnetic measurement markers are the preset number of markers set on the trunk of the subject during the measurement of the cardiac magnetic signals, positions of the preset number of body surface contrast markers are in one-to-one correspondence with positions of the preset number of cardiac magnetic measurement markers, and the preset number is at least 4;

aligning the first spatial coordinate with the second spatial coordinate based on the body surface contrast markers and the cardiac magnetic measurement markers with consistent positions to obtain a transformation matrix between the coordinate system for the dynamic structural information and the digital coordinate system;

calculating positions of the sensors acquiring the cardiac magnetic signals relative to the cardiac magnetic measurement markers to obtain positions of the sensors; and

obtaining the coordinates of the sensors in the coordinate system for the dynamic structural information based on the positions of the sensors and the transformation matrix.

According to the method for four-dimensional cardiac function imaging based on the biomagnetic signals provided in the present application, generating the dynamic distributed cardiac equivalent source model during the full cardiac cycle based on the plurality of frames of dynamic images includes:

determining a template frame from the plurality of frames of dynamic images;

registering systolic and diastolic images of the full cardiac cycle based on a similarity between the template frame and another plurality of frames of dynamic images to obtain a deformation field;

placing a grid point in a three-dimensional space of a target spatial range corresponding to the template frame and determining the grid point as a distributed cardiac equivalent source model of the template frame, where the target spatial range is a region within the pericardium and outside the cardiac chamber; and

adjusting the distributed cardiac equivalent source model of the template frame based on the deformation field to obtain the distributed cardiac equivalent source model corresponding to each frame of dynamic image, and to obtain the dynamic distributed cardiac equivalent source model during the full cardiac cycle.

According to the method for four-dimensional cardiac function imaging based on the biomagnetic signals provided in the present application, performing calculation and reconstruction on cardiac electrical excitation activity based on the cardiac magnetic signals and the dynamic lead field matrix to obtain the calculation and reconstruction results includes:

based on a minimum residual principle, constructing a cost function according to residual terms and regularization terms corresponding to a regularization constraint; where the residual terms are differences between the cardiac magnetic signals and simulated data obtained by performing forward calculation through the dynamic lead field matrix; and

minimizing the cost function to obtain activity changes of each reconstructed source in the source model as the calculation and reconstruction results.

According to the method for four-dimensional cardiac function imaging based on the biomagnetic signals provided in the present application, the four-dimensional visual image includes at least one of a cardiac current density distribution and change map, a cardiac activation-recovery map, or a dominant frequency distribution map of cardiac activities.

The present application further provides an apparatus for four-dimensional cardiac function imaging based on biomagnetic signals, including:

a structural information acquisition module configured to acquire dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images;

a cardiac magnetic signal acquisition module configured to acquire cardiac magnetic signals of a subject;

a three-dimensional spatial registration module configured to perform spatial registration on the dynamic structural information and sensors acquiring the cardiac magnetic signals to obtain coordinates of the sensors in a coordinate system for the dynamic structural information;

a data processing module configured to perform calculation and reconstruction on cardiac electrical excitation activity based on the dynamic structural information, the coordinates of the sensors, and the cardiac magnetic signals to obtain calculation and reconstruction results; and

a four-dimensional visualization module configured to convert the calculation and reconstruction results into a four-dimensional visual image.

The present application further provides an electronic device, including a memory, a processor, and a computer program stored on the memory and executable on the processor, where the processor, when executing the computer program, performs the method for four-dimensional cardiac function imaging based on biomagnetic signals described above.

The present application further provides a non-transitory computer-readable storage medium storing a computer program, where the computer program, when executed by a processor, performs the method for four-dimensional cardiac function imaging based on biomagnetic signals described above.

The present application further provides a computer product storing a computer program, where the computer program, when executed by a processor, performs the method for four-dimensional cardiac function imaging based on biomagnetic signals described above.

In a method and an apparatus for four-dimensional cardiac function imaging based on biomagnetic signals provided in the present application, by acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images; spatial registration is performed on the dynamic structural information and sensors acquiring the cardiac magnetic signals to obtain coordinates of the sensors in a coordinate system for the dynamic structural information; calculation and reconstruction are performed on cardiac electrical excitation activity based on the dynamic structural information, the coordinates of the sensors, and the cardiac magnetic signals to obtain calculation and reconstruction results; and the calculation and reconstruction results are converted into a four-dimensional visual image. In the present application, cardiac magnetic imaging is combined with cardiac motion. Based on the acquired dynamic structural information and the cardiac magnetic signals, the characteristics of biomagnetic signals are fully utilized. The spatial registration is integrated with signal processing technology to accurate reconstruct the four-dimensional cardiac representation and visually display functional activities. This results in a four-dimensional visual image comprehensively representing the dynamic electrophysiological activities of the entire heart (including the epicardium, endocardium, and intramural tissues) throughout the cardiac cycle, providing a novel solution for non-invasive imaging of cardiac electrical excitation activity.

BRIEF DESCRIPTION OF THE DRAWINGS

To illustrate solutions in the present application or the related art more clearly, the drawings needed to be used in the descriptions of the embodiments or the related art are briefly introduced below. The drawings in the following description are some embodiments of the present application, and other drawings can be obtained based on the drawings for those skilled in the art without any creative work.

FIG. 1 is a schematic flowchart of a method for four-dimensional cardiac function imaging based on biomagnetic signals according to the present application;

FIG. 2 is a schematic flowchart of constructing a dynamic distributed cardiac equivalent source model during a full cardiac cycle in a method for four-dimensional cardiac function imaging based on biomagnetic signals according to the present application;

FIG. 3 is a schematic diagram of a dynamic lead field matrix in a method for four-dimensional cardiac function imaging based on biomagnetic signals according to the present application;

FIG. 4 is a schematic diagram of calculation and reconstruction results in a method for four-dimensional cardiac function imaging based on biomagnetic signals according to the present application;

FIG. 5 is a first schematic diagram of a four-dimensional visual image in a method for four-dimensional cardiac function imaging based on biomagnetic signals according to the present application;

FIG. 6 is a second schematic diagram of a four-dimensional visual image in a method for four-dimensional cardiac function imaging based on biomagnetic signals according to the present application;

FIG. 7 is a third schematic diagram of a four-dimensional visual image in a method for four-dimensional cardiac function imaging based on biomagnetic signals according to the present application;

FIG. 8 is a schematic structural diagram of an apparatus for four-dimensional cardiac function imaging based on biomagnetic signals according to the present application; and

FIG. 9 is a schematic structural diagram of an electronic device according to the present application.

DETAILED DESCRIPTION

To illustrate objectives, solutions and advantages of the present application, the solutions of the present application are described clearly and completely below in conjunction with the drawings in the application. The described embodiments are part of the embodiments of the application, not all of them. All other embodiments obtained by those skilled in the art based on the embodiments of the present application without any creative work fall within the protection scope of the present application.

It is understood that cardiac magnetic imaging is a technique that uses the measurement of weak biological magnetic signals to achieve non-invasive imaging and visualization of cardiac electrical excitation activity, which has important guiding significance for diagnosis and treatment of heart diseases such as coronary heart disease, arrhythmia, and myocardial ischemia.

Cardiac electrical excitation activity determines cardiac physiological functions of blood-pumping and contraction. A complete cardiac cycle begins at the heart's pacemaker-sinoatrial nodes. Electrical signals from the sinoatrial nodes trigger electrical excitation in the atrial myocytes, leading to atrial contraction. During this process, blood in atria is effectively pumped into the ventricles. Subsequently, the electrical signals travel from the sinoatrial nodes to atrioventricular nodes located between the atria and ventricles, and rapidly and systematically spread then to both ventricles via Purkinje fibers. Upon receiving the electrical signals, the ventricular myocytes become excited, triggering a powerful ventricular contraction that propels blood from the heart into a systemic circulation. Finally, the ventricular myocytes undergo repolarization, returning to a resting state and preparing for the next cardiac cycle.

Electromagnetic theory indicates that current signals are always accompanied by magnetic field signals. Unlike electrocardiogram (ECG) signals, magnetic field signals are almost unaffected by the non-uniform conductivity of body tissues, making them more reliable in detecting biological phenomena. Furthermore, due to different physical properties of electric and magnetic fields, cardiac magnetic field signals are sensitive to tangential and eddy current sources, while electric signals are more sensitive to radial sources. The cardiac magnetic field signals may provide information that is difficult to obtain from ECGs or body surface potential maps. Therefore, non-invasive cardiac magnetic imaging can provide spatiotemporal information on cardiac electrical excitation activity, solving the problem of locating small lesions in typical cardiovascular diseases such as coronary heart disease and arrhythmias. It can be used for large-scale screening and auxiliary diagnosis of heart disease, and preoperative guidance for cardiac ablation surgery. The present application provides a method and an apparatus for four-dimensional cardiac function imaging based on biomagnetic signals.

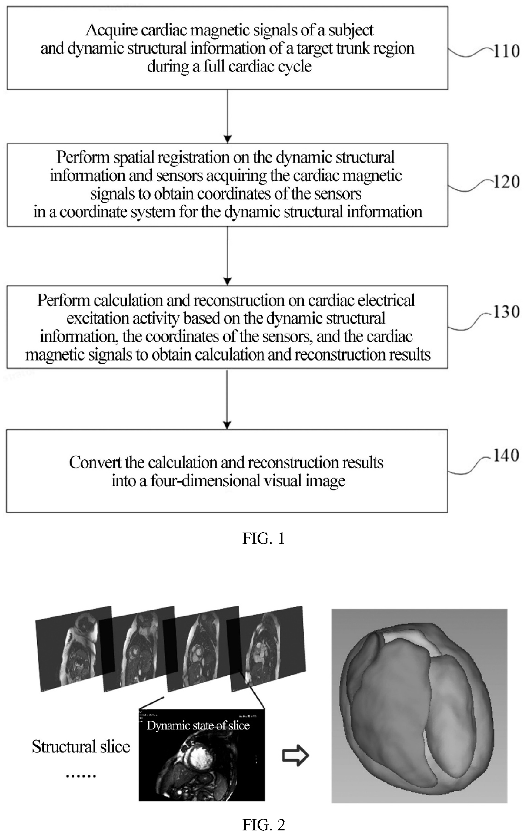

A method for four-dimensional cardiac function imaging based on biomagnetic signals according to the present application is described below in conjunction with FIG. 1 to FIG. 7. FIG. 1 is a schematic flowchart of a method for four-dimensional cardiac function imaging based on biomagnetic signals according to the present application. As shown in FIG. 1, the method includes following steps.

Step 110, acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images.

It should be noted that the acquired data includes multi-channel cardiac magnetic signals of the subject, as well as the contour structure and another plurality of frames of dynamic images of the target trunk region during the full cardiac cycle. The multi-channel cardiac magnetic signals are also known as cardiac magnetic signals, and the contour structure and a plurality of frames of dynamic images are collectively referred to as dynamic structural information. The target trunk region includes at least lungs, trunk, cardiac chambers, and pericardial structures of the subject.

The present application does not limit devices and methods for acquiring the dynamic structural information. In some embodiments, the dynamic structural information is acquired using any of magnetic resonance imaging (MRI), computed tomography (CT), echocardiography (EC), or a combination thereof.

Furthermore, in some embodiments, obtaining the dynamic structural information includes the following steps: acquiring a plurality of frames of dynamic images of the target trunk region of the subject with the full cardiac cycle, performing three-dimensional reconstruction on the images, segmenting and extracting a contour structure of the target trunk region based on three-dimensional reconstruction results, and finally obtaining the cardiac dynamic structural information.

The cardiac magnetic signals referred to in the present application are extracted using a distributed magnetic sensor array. The present application does not limit devices and methods for acquiring the cardiac magnetic signals. In some embodiments, the cardiac magnetic signals are acquired using any of the following methods: a magnetocardiometer based on a superconducting quantum interference device (SQUID), an optical pumping magnetometer (OPM) array, or an atomic magnetometer (AM) array. In an embodiment, the device for acquiring the cardiac magnetic signals is a 168-channel OPM array consisting of 84 biaxially oriented sensors arranged on all four sides, with 30 sensors on the front of the chest, 30 sensors on the back, and 12 sensors on left and right sides.

Furthermore, the subject referred to in the present application can be any living organism, including but not limited to mammals such as dogs and pigs, as well as non-human primates such as monkeys, and the present application does not limit thereto.

Step 120, performing spatial registration on the dynamic structural information and sensors acquiring the cardiac magnetic signals to obtain coordinates of the sensors in a coordinate system for the dynamic structural information.

Step 120 is intended to ensure that the spatial coordinates of the sensor extracting the cardiac magnetic signals are precisely aligned with the structural image of the dynamic structural information in a same spatial coordinate system. In the specific registration process, the trunk of the subject is marked during the acquisition of dynamic structural information and the acquisition of the cardiac magnetic signals. Spatial registration is performed based on the set marks, and then the coordinates of the sensors in the coordinate system for the dynamic structural information are obtained based on the registration result. The specific steps of spatial registration and obtaining the sensor's coordinates in the coordinate system for the dynamic structural information are further explained in subsequent embodiments of the present application.

Step 130, performing calculation and reconstruction on cardiac electrical excitation activity based on the dynamic structural information, the coordinates of the sensors, and the cardiac magnetic signals to obtain calculation and reconstruction results.

In step 130, the cardiac magnetic signal during the full cardiac cycle is dynamically reconstructed by combining reconstruction results of the cardiac electrical excitation activity (i.e., cardiac magnetic signals), the dynamic structural data of the subject (i.e., dynamic structural information), and the coordinates of the sensors.

Step 140, converting the calculation and reconstruction results into a four-dimensional visual image.

In step 140, cardiac functional activity data in the calculation and reconstruction results are converted into a four-dimensional image and visualized. Simultaneously, the four-dimensional visual image can be stored on a computing terminal and display device. It is important to emphasize that the generated four-dimensional visual image comprehensively covers the functional activity of the entire heart (including the epicardium, endocardium, and intramural tissues) throughout a complete cardiac cycle.

In the present application, the characteristics of biomagnetic signals are fully utilized and spatial registration is integrated with signal processing technology to accurate reconstruct the four-dimensional cardiac representation and visually display functional activities, which can not only capture static images of the heart structure but also dynamically display the functional changes of the heart during the cardiac cycle. This is of great significance for a deeper understanding of the electrophysiological characteristics of the heart and further observation of related cardiac regions.

Step 120 is further explained below. In some embodiments, performing spatial registration on the dynamic structural information and sensors acquiring the cardiac magnetic signals to obtain coordinates of the sensors in the coordinate system for the dynamic structural information includes:

step 121, marking a first spatial coordinate of each body surface contrast marker in the coordinate system for the dynamic structural information, and recording a second spatial coordinate of each cardiac magnetic measurement marker in a digital coordinate system using a digital measurement device; where body surface contrast markers are a preset number of markers set on a trunk of the subject during acquisition of the dynamic structural information, and the cardiac magnetic measurement markers are the preset number of markers set on the trunk of the subject during the measurement of the cardiac magnetic signals, positions of the preset number of body surface contrast markers are in one-to-one correspondence with positions of the preset number of cardiac magnetic measurement markers, and the preset number is at least 4;

step 122, aligning the first spatial coordinate with the second spatial coordinate based on the body surface contrast markers and the cardiac magnetic measurement markers with consistent positions to obtain a transformation matrix between the coordinate system for the dynamic structural information and the digital coordinate system;

step 123, calculating positions of the sensors acquiring the cardiac magnetic signals relative to the cardiac magnetic measurement markers to obtain positions of the sensors; and

step 124, obtaining the coordinates of the sensors in the coordinate system for the dynamic structural information based on the positions of the sensors and the transformation matrix.

It should be noted that to facilitate spatial registration, markers are set on the trunk of the subject during the acquisition of cardiac magnetic signals and dynamic structural information in step 110. During the acquisition of dynamic structural information, a preset number of markers are set on the trunk of the subject, and are designated as body surface contrast markers; during the acquisition of cardiac magnetic signals, a preset number of markers are set on the trunk of the subject, and are designated as cardiac magnetic measurement markers.

It should be noted that the number of body surface contrast markers are the same as the number of cardiac magnetic signals, and their positions are in one-to-one correspondence. In other words, a preset number of body surface contrast markers are in one-to-one correspondence with a preset number of cardiac magnetic measurement markers, and the corresponding body surface contrast markers and the cardiac magnetic measurement markers are located in the same position.

In some embodiments, the body surface contrast markers are made of grease, and the cardiac magnetic measurement marker are marked as coils.

Furthermore, it should be emphasized that the preset number is at least 4. In a specific embodiment, the preset number is 4.

In step 121, a first spatial coordinate of each body surface contrast marker in the coordinate system for the dynamic structural information is marked. It is understood that the coordinate system of dynamic structural information is typically the coordinate system of the device acquiring the dynamic structural information. For example, in a specific embodiment, if an MRI device is used to acquire dynamic structural information, then the coordinate system for the dynamic structural information is the MRI coordinate system. In this case, the first spatial coordinates of the body surface contrast markers can be marked manually.

While marking the first spatial coordinate of the body surface contrast markers, a digital measurement device is used to record the second spatial coordinate of each cardiac magnetic measurement marker in a digital coordinate system. It is emphasized that the digital coordinate system refers to a coordinate system of the used digital measurement device. For example, based on the above embodiment, if a marker coil is used as the cardiac magnetic measurement marker, an electromagnetic digitizer is used then to record the second spatial coordinate of the marker coil in the digitized coordinate system of the electromagnetic digitizer.

In step 122, the preset number of body surface contrast markers in the coordinate system for the dynamic structural information are aligned with the preset number of cardiac magnetic measurement markers in the digital coordinate system to obtain a transformation matrix between the two spatial coordinate systems. In an embodiment, the first spatial coordinate is aligned with second spatial coordinate using the body surface contrast markers and the cardiac magnetic measurement markers with consistent positions to minimize a matching error and to obtain a transformation matrix between the coordinate system for the dynamic structural information and the digital coordinate system. In some embodiments, the preset number of body surface contrast markers in the coordinate system for the dynamic structural information are aligned with the preset number of cardiac magnetic measurement markers in the digital coordinate system through a non-iterative least squares method.

In step 123, positions of the sensors acquiring the cardiac magnetic signals relative to the cardiac magnetic measurement markers are calculated to obtain positions of the sensors. In this step, positions of all the sensors may be obtained through measuring a position of a certain sensor relative to the cardiac magnetic measurement markers since relationships among relative positions of the sensors acquiring the cardiac magnetic signals in the distributed magnetic sensor array are determined. Based on the above embodiments, a marker coil is used as each cardiac magnetic measurement marker. Simultaneously, sine wave signals with a stable frequency are applied through a high-precision current source when the OPM device acquires the cardiac magnetic signals. Based on this, the magnetic field generated by the marker coil is used to calculate a position of the OPM sensor relative to the marker coil.

In step 124, coordinates of the sensors in the coordinate system for the dynamic structural information based on the positions of the sensors and the transformation matrix to complete the spatial registration.

In the embodiment of the present application, the sensors acquiring the cardiac magnetic signals and the dynamic structural information are placed in the same coordinate system using spatial registration technology, and more accurate spatiotemporal information on cardiac electrical excitation activity is provided through this non-invasive cardiac magnetic imaging, facilitating subsequent signal integration.

Step 130 is further explained below. In some embodiments, performing calculation and reconstruction on cardiac electrical excitation activity based on the dynamic structural information, the coordinates of the sensors, and the cardiac magnetic signals to obtain calculation and reconstruction results includes:

step 131, generating a dynamic distributed cardiac equivalent source model during a full cardiac cycle based on the plurality of frames of dynamic images;

step 132, performing calculation based on the dynamic distributed cardiac equivalent source model, the coordinates of the sensor, and the contour structure to obtain a dynamic lead field matrix; and

step 133, performing calculation and reconstruction on cardiac electrical excitation activity based on the cardiac magnetic signals and the dynamic lead field matrix to obtain the calculation and reconstruction results.

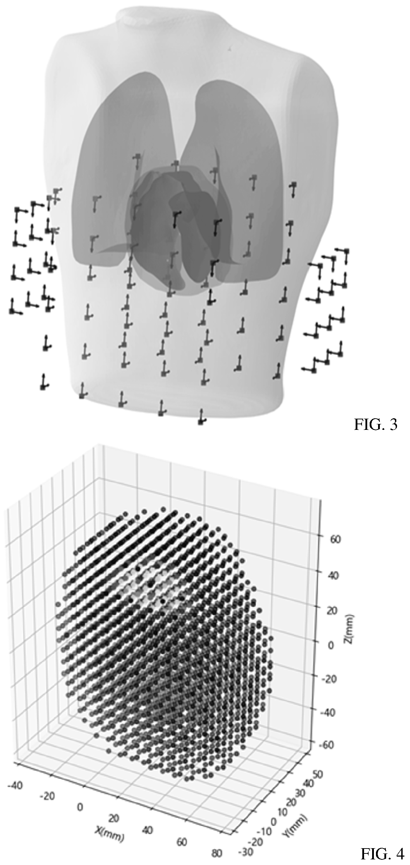

In step 131, as shown in FIG. 2, a distributed cardiac equivalent source model for each frame of the image is constructed using the plurality of frames of dynamic images to obtain a dynamic distributed cardiac equivalent source model during a full cardiac cycle. In step 132, on the basis of dynamic distributed cardiac equivalent source mode, calculation is performed using the coordinates of the sensor obtained in step 120 and the contour structure obtained in step 110 to obtain a dynamic lead field matrix based on the obtained dynamic distributed cardiac equivalent source model (as shown in FIG. 3). In some embodiments, calculation is performed using a boundary element method or a finite element method to obtain the dynamic lead field matrix mentioned above.

In an embodiment, calculation is performed using a boundary element method to obtain the dynamic lead field matrix mentioned above. The boundary curve surface is discretized into a finite number of triangular surface elements, and the theoretical mathematical physics calculation equations for calculating the magnetic field from source activity, obtained from Maxwell's equations, are transformed into a system of linear algebraic equations, obtaining the lead field matrix.

In the calculations, the cardiac conductivity was taken as 0.0537 to 0.483 S/m. The conductivity of the lungs is taken as 0.5 S/ m, and the conductivity of the trunk is taken as 0.216-0.241 S/m. The conductivity inside the cardiac chamber is taken as 0.4-1.0 S/m.

In step 133, calculation and reconstruction are performed on cardiac electrical excitation activity based on the cardiac magnetic signals and the dynamic lead field matrix to obtain the calculation and reconstruction results. It should be noted that the calculation and reconstruction results include the activity changes after each source in the source model is reconstructed. In some embodiments, calculation and reconstruction are performed on the cardiac electrical excitation activity using a regularization method.

In the present application, the characteristics of biomagnetic signals are fully utilized, the spatial registration is integrated with signal processing technology to perform calculation and reconstruction on dynamic three-dimensional sources of cardiac magnetic signals during the full cardiac cycle.

Step 131 is further explained below. In some embodiments, generating a dynamic distributed cardiac equivalent source model during a full cardiac cycle based on the plurality of frames of dynamic images includes:

  step 1311, determining a template frame from the plurality of frames of dynamic images;

  step 1312, registering systolic and diastolic images of the full cardiac cycle based on a similarity between the template frame and another plurality of frames of dynamic images to obtain a deformation field;

  step 1313, placing a grid point in a three-dimensional space of a target spatial range corresponding to the template frame and determining the grid point as a distributed cardiac equivalent source model of the template frame, where the target spatial range is a region within the pericardium and outside the cardiac chamber; and

  step 1314, adjusting the distributed cardiac equivalent source model of the template frame based on the deformation field to obtain the distributed cardiac equivalent source model corresponding to each frame of dynamic image, and to obtain the dynamic distributed cardiac equivalent source model during the full cardiac cycle.

In step 1311, a template frame is determined from the plurality of frames of dynamic images obtained in step 110. The template frame can be any frame from the plurality of frames of dynamic images. In an embodiment, a segmented first frame of image is used as the template frame.

In step 1312, systolic and diastolic images of the full cardiac cycle are registered with based on the template frame to obtain a deformation field. During registration, the similarity between the template frame and other multiple dynamic images is used. In an implementation, the registration is performed based on the similarity between the template frame and other multiple dynamic images through various methods, such as a torque and principal axis-based registration method, an intensity difference and associated registration method, and a mutual information-based registration method.

The torque and principal axis-based registration method includes performing similarity calculation using statistical factors derived from image data and then performing image registration. Torque describes the spatial distribution of image quality (intensity). A torque-based registration method includes performing image registration by overlapping the torques in the image while a principal axis-based method includes performing image registration by overlapping principal axes of the inertia tensors of corresponding objects in the image.

The intensity difference and associated registration method attempts to determine an optimal registration scheme by maximizing the similarity between different images, which are mainly due to different image acquisition conditions (such as noises). These methods typically assume that the pixel values in the registered images are strongly associated.

Mutual information, as referred to in the mutual information-based registration method, is an information-theoretic metric used to measure the statistical dependence between two random variables, or the amount of information a variable contains about another variable. Mutual information can be qualitatively viewed as a measure of how well one image explains another. Mutual information is maximized for optimal alignment.

Furthermore, to facilitate calculation, similarity calculation can be performed by extracting only the most distinct image voxels from the plurality of frames of dynamic images.

In step 1313, a grid point is placed in a three-dimensional space (a target spatial range) within the pericardium and outside the cardiac chamber corresponding to the template frame and determined as a distributed cardiac equivalent source model of the template frame. It should be noted that the present application does not limit the grid spacing, which can be set according to computational efficiency. However, it is understood that setting the grid spacing too large is inappropriate. In an embodiment, the grid spacing is 5 mm.

In step 1314, the distributed cardiac equivalent source model of the template frame is adjusted based on deformation field to obtain the distributed cardiac equivalent source model corresponding to each frame of image, and the distributed cardiac equivalent source models corresponding to all frames of dynamic images collectively constitute the dynamic distributed cardiac equivalent source model during the full cardiac cycle.

In the embodiment of the present application, the plurality of frames of dynamic images are converted the dynamic distributed cardiac equivalent source model during the full cardiac cycle, serving as the basis for signal integration.

Step 133 is further explained below. In some embodiments, performing calculation and reconstruction on the cardiac electrical excitation activity based on the cardiac magnetic signals and the dynamic lead field matrix to obtain the calculation and reconstruction results includes:

based on a minimum residual principle, constructing a cost function according to residual terms and regularization terms corresponding to a regularization constraint; where the residual terms are differences between the cardiac magnetic signals and simulated data obtained by performing forward calculation through the dynamic lead field matrix; and

minimizing the cost function to obtain activity changes of each source in the source model after being reconstructed as the calculation and reconstruction results.

In an embodiment, calculation and reconstruction is performed on the cardiac electrical excitation activity by minimizing the cost function based on the cardiac magnetic signals obtained in step 110 and the dynamic lead field matrix obtained in step 132, to obtain activity changes of each reconstructed source in the source model as the calculation and reconstruction results. In an embodiment, the calculation and reconstruction results are shown in FIG. 4.

It should be explained that the cost function is constructed by combining residual terms, which are differences between measurement data of the cardiac magnetic signals and the simulated data obtained by performing forward calculation through the dynamic lead field matrix, and regularization terms corresponding to the regularization constraint, based on the minimum residual principle.

In an embodiment, the cost function is represented by a first preset formula.

The first preset formula is

$$L(S) = ||X - GS||_F^Z + \lambda f(S),$$

where $L(S)$ is a cost function for solving the inverse problem, and the solution to the inverse problem can be obtained by minimizing $L(S)$; $|| \ ||_F^Z$ represents the F-norm of the data, $X$ is a matrix of measurement data of the cardiac magnetic signals, $G$ is a forward lead field matrix obtained by the finite element method or the boundary element method, $S$ is a distributed matrix of a source to be solved, $\lambda$ represents a regularization parameter, and $f(S)$ represents regularization terms (generally a noise covariance matrix).

In the embodiment of the present application, spatial registration is integrated with signal processing technology to accurately reconstruct the cardiac four-dimensional representation through fully utilizing the characteristics of biomagnetic signals.

Furthermore, in some embodiments, the four-dimensional visual image includes at least one of a cardiac current density distribution and change map, a cardiac activation-recovery map, or a dominant frequency distribution map of cardiac activities.

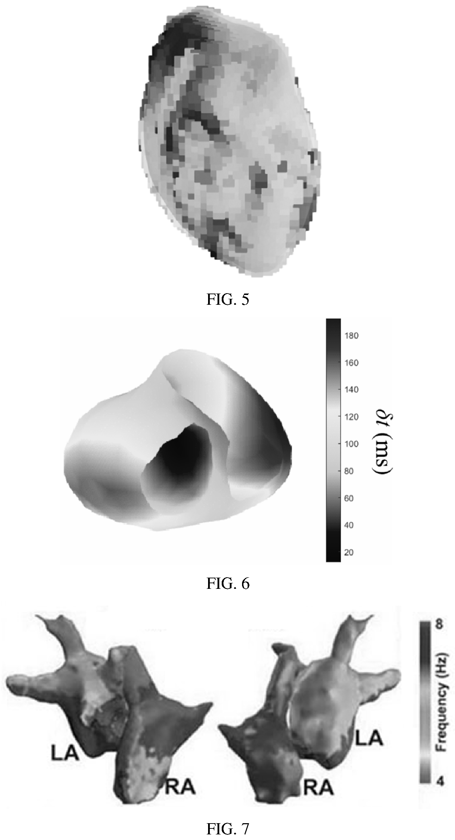

The four-dimensional visual image may include a cardiac current density distribution and change map, as shown in FIG. 5, where different colors represent different activation intensities. The four-dimensional visual image may further include a cardiac activation-recovery map, as shown in FIG. 6, where different colors represent different activation times. The four-dimensional visual image may further include a dominant frequency distribution map of cardiac activities, as shown in FIG. 7, where different colors represent different activation response frequencies.

The present application provides a method and an apparatus for four-dimensional cardiac function imaging based on biomagnetic signals. By acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images; spatial registration is performed on the dynamic structural information and sensors acquiring the cardiac magnetic signals to obtain coordinates of the sensors in a coordinate system for the dynamic structural information; calculation and reconstruction are performed on cardiac electrical excitation activity based on the dynamic structural information, the coordinates of the sensors, and the cardiac magnetic signals to obtain calculation and reconstruction results; and the calculation and reconstruction results are converted into a four-dimensional visual image. In the present application, cardiac magnetic imaging is combined with cardiac motion. Based on the acquired dynamic structural information and the cardiac magnetic signals, the characteristics of biomagnetic signals are fully utilized. The spatial registration is integrated with signal processing technology to accurate reconstruct the four-dimensional cardiac representation and visually display functional activities. This results in a four-dimensional visual image comprehensively representing the dynamic electrophysiological activities of the entire heart (including the epicardium, endocardium, and intramural tissues) throughout the cardiac cycle, providing a novel solution for non-invasive imaging of cardiac electrical excitation activity.

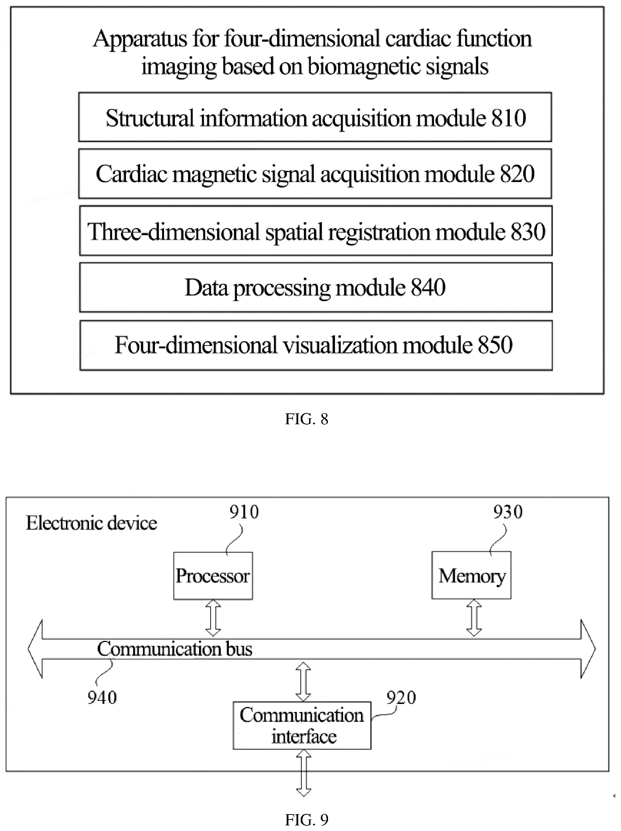

An apparatus for four-dimensional cardiac function imaging based on the biomagnetic signals is described below and the apparatus for four-dimensional cardiac function imaging based on the biomagnetic signals described below is referred to the method for four-dimensional cardiac function imaging based on the biomagnetic signals described above. As shown in FIG. 8, the apparatus includes:

> a structural information acquisition module 810 configured to acquire dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images;

> a cardiac magnetic signal acquisition module 820 configured to acquire cardiac magnetic signals of a subject;

> a three-dimensional spatial registration module 830 configured to perform spatial registration on the dynamic structural information and sensors acquiring the cardiac magnetic signals to obtain coordinates of the sensors in a coordinate system for the dynamic structural information;

> a data processing module 840 configured to perform calculation and reconstruction on cardiac electrical excitation activity based on the dynamic structural information, the coordinates of the sensors, and the cardiac magnetic signals to obtain calculation and reconstruction results; and

> a four-dimensional visualization module 850 configured to convert the calculation and reconstruction results into a four-dimensional visual image.

According to the apparatus for four-dimensional cardiac function imaging based on the biomagnetic signals provided in the present application, performing the calculation and reconstruction on cardiac electrical excitation activity based on the dynamic structural information, the coordinates of the sensors, and the cardiac magnetic signals to obtain the calculation and reconstruction results includes:

> generating a dynamic distributed cardiac equivalent source model during a full cardiac cycle based on the plurality of frames of dynamic images;

> performing calculation based on the dynamic distributed cardiac equivalent source model, the coordinates of the sensor, and the contour structure to obtain a dynamic lead field matrix; and

> performing calculation and reconstruction on cardiac electrical excitation activity based on the cardiac magnetic signals and the dynamic lead field matrix to obtain the calculation and reconstruction results.

According to the apparatus for four-dimensional cardiac function imaging based on the biomagnetic signals provided in the present application, performing the spatial registration on the dynamic structural information and the sensors acquiring the cardiac magnetic signals to obtain the coordinates of the sensors in the coordinate system for the dynamic structural information includes:

> marking a first spatial coordinate of each body surface contrast marker in the coordinate system for the dynamic structural information, and recording a second spatial coordinate of each cardiac magnetic measurement marker in a digital coordinate system using a digital measurement device; where body surface contrast markers are a preset number of markers set on a trunk of the subject during acquisition of the dynamic structural information, and the cardiac magnetic measurement markers are the preset number of markers set on the trunk of the subject during the measurement of the cardiac magnetic signals, positions of the preset number of body surface contrast markers are in one-to-one correspondence with positions of the preset number of cardiac magnetic measurement markers, and the preset number is at least 4;

> aligning the first spatial coordinate with the second spatial coordinate based on the body surface contrast markers and the cardiac magnetic measurement markers with consistent positions to obtain a transformation matrix between the coordinate system for the dynamic structural information and the digital coordinate system;

> calculating positions of the sensors acquiring the cardiac magnetic signals relative to the cardiac magnetic measurement markers to obtain positions of the sensors; and

obtaining the coordinates of the sensors in the coordinate system for the dynamic structural information based on the positions of the sensors and the transformation matrix.

According to the apparatus for four-dimensional cardiac function imaging based on the biomagnetic signals provided in the present application, generating the dynamic distributed cardiac equivalent source model during the full cardiac cycle based on the plurality of frames of dynamic images includes:

determining a template frame from the plurality of frames of dynamic images;

registering systolic and diastolic images of the full cardiac cycle based on a similarity between the template frame and another plurality of frames of dynamic images to obtain a deformation field;

placing a grid point in a three-dimensional space of a target spatial range corresponding to the template frame and determining the grid point as a distributed cardiac equivalent source model of the template frame, where the target spatial range is a region within the pericardium and outside the cardiac chamber; and

adjusting the distributed cardiac equivalent source model of the template frame based on the deformation field to obtain the distributed cardiac equivalent source model corresponding to each frame of dynamic image, and to obtain the dynamic distributed cardiac equivalent source model during the full cardiac cycle.

According to the apparatus for four-dimensional cardiac function imaging based on the biomagnetic signals provided in the present application, performing calculation and reconstruction on cardiac electrical excitation activity based on the cardiac magnetic signals and the dynamic lead field matrix to obtain the calculation and reconstruction results includes:

based on a minimum residual principle, constructing a cost function according to residual terms and regularization terms corresponding to a regularization constraint; where the residual terms are differences between the cardiac magnetic signals and simulated data obtained by performing forward calculation through the dynamic lead field matrix; and

minimizing the cost function to obtain activity changes of each reconstructed source in the source model as the calculation and reconstruction results.

According to the apparatus for four-dimensional cardiac function imaging based on the biomagnetic signals provided in the present application, the four-dimensional visual image includes at least one of a cardiac current density distribution and change map, a cardiac activation-recovery map, or a dominant frequency distribution map of cardiac activities.

In an apparatus for four-dimensional cardiac function imaging based on biomagnetic signals provided in the present application, by acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images; spatial registration is performed on the dynamic structural information and sensors acquiring the cardiac magnetic signals to obtain coordinates of the sensors in a coordinate system for the dynamic structural information; calculation and reconstruction are performed on cardiac electrical excitation activity based on the dynamic structural information, the coordinates of the sensors, and the cardiac magnetic signals to obtain calculation and reconstruction results; and the calculation and reconstruction results are converted into a four-dimensional visual image. In the present application, cardiac magnetic imaging is combined with cardiac motion. Based on the acquired dynamic structural information and the cardiac magnetic signals, the characteristics of biomagnetic signals are fully utilized. The spatial registration is integrated with signal processing technology to accurate reconstruct the four-dimensional cardiac representation and visually display functional activities. This results in a four-dimensional visual image comprehensively representing the dynamic electrophysiological activities of the entire heart (including the epicardium, endocardium, and intramural tissues) throughout the cardiac cycle, providing a novel solution for non-invasive imaging of cardiac electrical excitation activity.

FIG. 9 is a schematic structural diagram of an electronic device. As shown in FIG. 9, the electronic device may include a processor 910, a communication interface 920, a memory 930, and a communication bus 940. The processor 910, the communication interface 920, and the memory 930 communicate with each other through the communication bus 940. The processor (910) can call logical instructions in the memory (930) to perform a method for four-dimensional cardiac function imaging based on biomagnetic signals. The method includes: acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images; performing spatial registration on the dynamic structural information and sensors acquiring the cardiac magnetic signals to obtain coordinates of the sensors in a coordinate system for the dynamic structural information; performing calculation and reconstruction on cardiac electrical excitation activity based on the dynamic structural information, the coordinates of the sensors, and the cardiac magnetic signals to obtain calculation and reconstruction results; and converting the calculation and reconstruction results into a four-dimensional visual image.

In addition, the logic instruction in the memory 930 described above may be implemented in the form of a software functional unit and may be stored in a computer readable storage medium while being sold or used as a separate product. Based on such understanding, the solutions of the present application in essence or a part of the solutions that contributes to the related art, or a part of the solutions, may be embodied in the form of a software product, which is stored in a storage medium, including several instructions to cause a computer device (which may be a personal computer, server, or network device, etc.) to perform all or part of steps of the methods described in the respective embodiments of the present application. The storage medium described above includes various media that may store program codes such as U disk, mobile hard disk, read-only memory (ROM), random access memory (RAM), magnetic disk, or a compact disk.

The present application further provides a computer program product, including a computer program that can be stored on a non-transitory computer-readable storage medium. A computer can perform a method for four-dimensional cardiac function imaging based on the biomagnetic signals provided by the above methods when the computer program executed by a processor. The method includes: acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images; performing spatial registration on the dynamic structural information and sensors acquiring the cardiac magnetic signals to obtain coordinates of the sensors in a coordinate system for the dynamic structural information; performing calculation and reconstruction on cardiac electrical excitation activity based on the dynamic structural information, the coordinates of the sensors, and the cardiac magnetic signals to obtain calculation and reconstruction results; and converting the calculation and reconstruction results into a four-dimensional visual image.

The present application further provides a non-transitory computer-readable storage medium storing a computer program thereon. the computer program, when executed by a processor, is executed to perform the method for four-dimensional cardiac function imaging based on the biomagnetic signals provided by the methods described above. The method includes: acquiring cardiac magnetic signals of a subject and dynamic structural information of a target trunk region during a full cardiac cycle, where the target trunk region includes at least two lungs, a trunk and cardiac chambers and a pericardial structure, and the dynamic structural information includes a contour structure and a plurality of frames of dynamic images; performing spatial registration on the dynamic structural information and sensors acquiring the cardiac magnetic signals to obtain coordinates of the sensors in a coordinate system for the dynamic structural information; performing calculation and reconstruction on cardiac electrical excitation activity based on the dynamic structural information, the coordinates of the sensors, and the cardiac magnetic signals to obtain calculation and reconstruction results; and converting the calculation and reconstruction results into a four-dimensional visual image.

The apparatus embodiments described above are merely illustrative, wherein the units described as separate components may or may not be physically separate, and the components displayed as units may or may not be physical units, that is, may be located at the same place or be distributed to multiple network units. Some or all of the modules may be selected according to actual needs to achieve the purpose of the solution of the present embodiment. Those skilled in the art can understand and implement the embodiments described above without paying creative work.

Through the description of the embodiments above, those skilled in the art can clearly understand that the various embodiments can be implemented by means of software and a necessary general hardware platform, and of course, by hardware. Based on such understanding, the solutions of the present application in essence or a part of the solutions that contributes to the prior art, or a part of the solutions, may be embodied in the form of a software product, which may be stored in a storage medium such as ROM/RAM, magnetic discs, optical discs, etc., and includes several instructions to cause a computer device (which may be a personal computer, server, or network device, etc.) to perform the methods described in various embodiments or a part thereof.

Finally, it should be noted that the above embodiments are only used to explain the solutions of the present application, and are not limited thereto; although the present application has been described in detail with reference to the foregoing embodiments, it should be understood by those skilled in the art that they can still modify the solutions documented in the foregoing embodiments and make equivalent substitutions to a part of the features; these modifications and substitutions do not make the essence of the corresponding solutions depart from the scope of the solutions of various embodiments of the present application.

## Claims

1. A method for four-dimensional cardiac function imaging based on biomagnetic signals, comprising:

   acquiring cardiac magnetic signals of a subject and dynamic structural information of a target trunk region during a full cardiac cycle, wherein the target trunk region comprises at least two lungs, a trunk and cardiac chambers and a pericardial structure, and the dynamic structural information comprises a contour structure and a plurality of frames of dynamic images;
   performing spatial registration on the dynamic structural information and sensors acquiring the cardiac magnetic signals to obtain coordinates of the sensors in a coordinate system for the dynamic structural information;
   performing calculation and reconstruction on cardiac electrical excitation activity based on the dynamic structural information, the coordinates of the sensors, and the cardiac magnetic signals to obtain calculation and reconstruction results; and
   converting the calculation and reconstruction results into a four-dimensional visual image.

2. The method of claim 1, wherein performing the calculation and reconstruction on cardiac electrical excitation activity based on the dynamic structural information, the coordinates of the sensors, and the cardiac magnetic signals to obtain the calculation and reconstruction results comprises:

   generating a dynamic distributed cardiac equivalent source model during a full cardiac cycle based on the plurality of frames of dynamic images;
   performing calculation based on the dynamic distributed cardiac equivalent source model, the coordinates of the sensor, and the contour structure to obtain a dynamic lead field matrix; and
   performing calculation and reconstruction on cardiac electrical excitation activity based on the cardiac magnetic signals and the dynamic lead field matrix to obtain the calculation and reconstruction results.

3. The method of claim 1, wherein performing the spatial registration on the dynamic structural information and the sensors acquiring the cardiac magnetic signals to obtain the coordinates of the sensors in the coordinate system for the dynamic structural information comprises:

   marking a first spatial coordinate of each body surface contrast marker in the coordinate system for the dynamic structural information, and recording a second spatial coordinate of each cardiac magnetic measurement marker in a digital coordinate system using a digital measurement device; wherein body surface contrast markers are a preset number of markers set on a trunk of the subject during acquisition of the dynamic structural information, and the cardiac magnetic measurement markers are the preset number of markers set on the trunk of the subject during the measurement of the cardiac magnetic signals, positions of the preset number of body surface contrast markers are in one-to-one correspondence with positions of the preset number of cardiac magnetic measurement markers, and the preset number is at least 4;
   aligning the first spatial coordinate with the second spatial coordinate based on the body surface contrast markers and the cardiac magnetic measurement markers with consistent positions to obtain a transformation matrix between the coordinate system for the dynamic structural information and the digital coordinate system;
   calculating positions of the sensors acquiring the cardiac magnetic signals relative to the cardiac magnetic measurement markers to obtain positions of the sensors; and
   obtaining the coordinates of the sensors in the coordinate system for the dynamic structural information based on the positions of the sensors and the transformation matrix.

4. The method of claim 2, wherein generating the dynamic distributed cardiac equivalent source model during the full cardiac cycle based on the plurality of frames of dynamic images comprises:

   determining a template frame from the plurality of frames of dynamic images;
   registering systolic and diastolic images of the full cardiac cycle based on a similarity between the template frame and another plurality of frames of dynamic images to obtain a deformation field;
   placing a grid point in a three-dimensional space of a target spatial range corresponding to the template frame and determining the grid point as a distributed cardiac equivalent source model of the template frame, wherein the target spatial range is a region within the pericardium and outside the cardiac chamber; and
   adjusting the distributed cardiac equivalent source model of the template frame based on the deformation field to obtain the distributed cardiac equivalent source model corresponding to each frame of dynamic image, and to obtain the dynamic distributed cardiac equivalent source model during the full cardiac cycle.

5. The method of claim 2, wherein performing calculation and reconstruction on cardiac electrical excitation activity based on the cardiac magnetic signals and the dynamic lead field matrix to obtain the calculation and reconstruction results comprises:

based on a minimum residual principle, constructing a cost function according to residual terms and regularization terms corresponding to a regularization constraint; the residual terms being differences between the cardiac magnetic signals and simulated data obtained by performing forward calculation through the dynamic lead field matrix; and
minimizing the cost function to obtain activity changes of each reconstructed source in the source model as the calculation and reconstruction results.

6. The method of claim 1, wherein the four-dimensional visual image comprises at least one of a cardiac current density distribution and change map, a cardiac activation-recovery map, or a dominant frequency distribution map of cardiac activities.

7. An apparatus for four-dimensional cardiac function imaging based on biomagnetic signals, comprising:

a structural information acquisition module configured to acquire dynamic structural information of a target trunk region during a full cardiac cycle, wherein the target trunk region comprises at least two lungs, a trunk and cardiac chambers and a pericardial structure, and the dynamic structural information comprises a contour structure and a plurality of frames of dynamic images;
a cardiac magnetic signal acquisition module configured to acquire cardiac magnetic signals of a subject;
a three-dimensional spatial registration module configured to perform spatial registration on the dynamic structural information and sensors acquiring the cardiac magnetic signals to obtain coordinates of the sensors in a coordinate system for the dynamic structural information;
a data processing module configured to perform calculation and reconstruction on cardiac electrical excitation activity based on the dynamic structural information, the coordinates of the sensors, and the cardiac magnetic signals to obtain calculation and reconstruction results; and
a four-dimensional visualization module configured to convert the calculation and reconstruction results into a four-dimensional visual image.

8. An electronic device comprising a memory, a processor, and a computer program stored on the memory and executable on the processor, wherein the processor, when executing the computer program, performs the method for four-dimensional cardiac function imaging based on biomagnetic signals of any of claims 1 to 6.

9. A non-transitory computer-readable storage medium storing a computer program, wherein the computer program, when executed by a processor, performs the method for four-dimensional cardiac function imaging based on biomagnetic signals of any of claims 1 to 6.

10. A computer product, comprising a computer program, wherein the computer program, when executed by a processor, performs the method for four-dimensional cardiac function imaging based on biomagnetic signals of any of claims 1 to 6.

Acquire cardiac magnetic signals of a subject
and dynamic structural information of a target trunk region
during a full cardiac cycle — 110

Perform spatial registration on the dynamic structural
information and sensors acquiring the cardiac magnetic
signals to obtain coordinates of the sensors
in a coordinate system for the dynamic structural information — 120

Perform calculation and reconstruction on cardiac electrical
excitation activity based on the dynamic structural
information, the coordinates of the sensors, and the cardiac
magnetic signals to obtain calculation and reconstruction results — 130

Convert the calculation and reconstruction results
into a four-dimensional visual image — 140

FIG. 1

Structural slice

Dynamic state of slice

......

FIG. 2

FIG. 3

FIG. 4

FIG. 5

FIG. 6

FIG. 7

Apparatus for four-dimensional cardiac function
imaging based on biomagnetic signals

Structural information acquisition module 810

Cardiac magnetic signal acquisition module 820

Three-dimensional spatial registration module 830

Data processing module 840

Four-dimensional visualization module 850

FIG. 8

Electronic device  910  930

Processor

Memory

Communication bus

940

Communication
interface  920

FIG. 9

### INTERNATIONAL SEARCH REPORT

International application No.

**PCT/CN2025/097299**

| A. | CLASSIFICATION OF SUBJECT MATTER |
| --- | --- |

A61B5/243(2021.01)i; A61B5/00(2006.01)i

According to International Patent Classification (IPC) or to both national classification and IPC

| B. | FIELDS SEARCHED |
| --- | --- |

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

WPABSC; CNTXT; ENTXTC; VEN; ENTXT; CNKI; 百度学术, BAIDU SCHOLAR; Web of Science: 南京景瑞康分子医药科技有限公司, 赵婷, 心磁, 心脏磁, 磁传感器, 心动周期, 坐标, 位置, 动态, 时间, 配准, 对齐, 标记, 标志, 标识, 四维, 4D, OPM, MCG, MEG, Magnetocardiography, Magnetoencephalography, optically pumped magnetometer, cardiac cycle, coordinates, location, dynamics, time, registration, alignment, marker, landmark, four dimensions

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CN 107708540 A (GENETESIS LLC) 16 February 2018 (2018-02-16) description, paragraphs [0004]-[0096], and figures 1-11 | 1-10 |
| Y | CN 117017300 A (BEIHANG UNIVERSITY) 10 November 2023 (2023-11-10) description, paragraphs [0005]-[0045], and figure 1 | 1-10 |
| Y | CN 115334962 A (ASAHI INTECC CO., LTD.) 11 November 2022 (2022-11-11) description, paragraphs [0005]-[0127], and figures 1-18 | 1-10 |
| Y | CN 118141380 A (BEIHANG UNIVERSITY) 07 June 2024 (2024-06-07) description, paragraphs [0005]-[0057], and figures 1-5 | 1-10 |
| Y | CN 117493812 A (BEIHANG UNIVERSITY) 02 February 2024 (2024-02-02) description, paragraphs [0005]-[0035], and figure 1 | 1-10 |
| A | US 2016220311 A1 (SIEMENS HEALTHCARE GMBH) 04 August 2016 (2016-08-04) entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 August 2025** | **20 August 2025** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2025/097299**

## C. DOCUMENTS CONSIDERED TO BE RELEVANT

| Category* | Citation of document, with indication, where appropriate, of the relevant passages | Relevant to claim No. |
|-----------|-----------------------------------------------------------------------------------|-----------------------|
| A | JP 2023145063 A (TDK CORP.) 11 October 2023 (2023-10-11) <br> entire document | 1-10 |
| A | CN 112842343 A (BEIHANG UNIVERSITY) 28 May 2021 (2021-05-28) <br> entire document | 1-10 |
| A | CN 117414138 A (BEIHANG UNIVERSITY) 19 January 2024 (2024-01-19) <br> entire document | 1-10 |
| A | JP H10323335 A (TOSHIBA CORP.) 08 December 1998 (1998-12-08) <br> entire document | 1-10 |

Form PCT/ISA/210 (second sheet) (July 2022)

**INTERNATIONAL SEARCH REPORT**
Information on patent family members

International application No.

**PCT/CN2025/097299**

| Patent document cited in search report | | | Publication date (day/month/year) | Patent family member(s) | | | Publication date (day/month/year) |
|---|---|---|---|---|---|---|---|
| CN | 107708540 | A | 16 February 2018 | None | | | |
| CN | 117017300 | A | 10 November 2023 | None | | | |
| CN | 115334962 | A | 11 November 2022 | None | | | |
| CN | 118141380 | A | 07 June 2024 | None | | | |
| CN | 117493812 | A | 02 February 2024 | None | | | |
| US | 2016220311 | A1 | 04 August 2016 | US | 10485510 | B2 | 26 November 2019 |
| JP | 2023145063 | A | 11 October 2023 | None | | | |
| CN | 112842343 | A | 28 May 2021 | None | | | |
| CN | 117414138 | A | 19 January 2024 | None | | | |
| JP | H10323335 | A | 08 December 1998 | JP | 3660781 | B2 | 15 June 2005 |

Form PCT/ISA/210 (patent family annex) (July 2022)

**EP 4 767 940 A1**

**Patent documents cited in the description**

- CN 202411058622X